# EUROPEAN PATENT APPLICATION

(11) **EP 4 488 362 A1**
(43) Date of publication of application: **08.01.2025**
(21) Application number: 23763380.5
(22) Date of filing: 27.02.2023
(51) Int. Cl.: C12N 5/071, A61K 35/12, A61L 27/38, A61P 17/02, A61P 25/00, C12P 21/02

(54) **GROWTH FACTOR-PRODUCING CELLS AND METHOD FOR PRODUCING SAME**

(30) Priority: 01.03.2022 JP 2022030908
(71) Applicant: Kataoka Corporation, Kyoto-shi, Kyoto 601-8203 (JP); KYOTO PREFECTURAL PUBLIC UNIVERSITY CORPORATION, Kyoto-shi, Kyoto 602-8566 (JP)
(72) Inventor: DAI, Ping, Kyoto-shi, Kyoto 602-8566 (JP); HARADA, Yoshinori, Kyoto-shi, Kyoto 602-8566 (JP); KURAHASHI, Toshihiro, Kyoto-shi, Kyoto 602-8566 (JP); NAMBA, Hiroko, Kyoto-shi, Kyoto 601-8203 (JP); MATSUMOTO, Junichi, Kyoto-shi Kyoto 601-8203 (JP)
(74) Representative: Vossius & Partner Patentanwälte Rechtsanwälte mbB
(86) International application number: PCT/JP2023/006927
(87) International publication number: WO 2023/167122

(57) **Abstract**

The present invention primarily aims to provide a new method to safely and rapidly induce or convert growth factor-producing cells from somatic cells without the need for gene transfer.

The present invention includes, for example, a method for producing growth factor-producing cells by inducing differentiation directly from somatic cells, comprising a step of culturing a somatic cell in the presence of at least any one selected from the group consisting of a TGF-β inhibitor, a p53 inhibitor, and a cAMP inducer.

The growth factor-producing cells obtained by the present invention are useful in regenerative medicine and other applications.

## Description

### Technical Field

### (Cross-reference to related applications)

The present application claims the benefit of Japanese Application No. 2022-30908, filed March 1, 2022 with the Japan Patent Office. The Japanese application is hereby incorporated by reference for all purposes as if the entirety of its application documents (specification, claims, drawings, and abstract) were expressly set forth herein.

The present invention relates primarily to a method for producing a growth factor-producing cell from a somatic cell by means of so-called low molecular weight compounds (e.g., compounds with a molecular weight of 1000 or less), and to a growth factor-producing cell produced by such a method. The present invention further relates to a method for producing a growth factor from the said growth factor-producing cell and a composition that can be used for the method for producing a growth factor-producing cell.

### Background of the Invention

Recent developments in cell-related research, particularly with respect to pluripotent cells, are making it possible to obtain therapeutic cells in quality and quantity that can be used for transplantation into individuals. For some diseases, attempts have been initiated to apply therapeutically effective cells to patients. For example, Non-Patent Document 1 examines an attempt at regenerative medicine for patients with subacute spinal cord injury by transplantation of induced pluripotent stem cell (iPS cell)-derived neural progenitor cells.

HGF (Hepatocyte Growth Factor), a type of growth factor, was originally discovered as a growth factor for hepatocytes. However, numerous recent studies have found that it also has angiogenic and neurotrophic effects in the central nervous system. For example, in Non-Patent Document 2, it is reported that HGF administration to a primate spinal cord injury model significantly promoted the recovery of motor function of the extremities. In addition, Patent Document 1 discloses a spinal cord injury therapeutic agent that contains HGF protein as an active ingredient.

### Prior Art

### Patent Document

Patent Document 1: International Publication No. 2008/105507 pamphlet. Non-Patent Document
Non-Patent Document 1: O. Tsuji et al, Stem Cells. 2019;37(1): 6-13.
Non-Patent Document 2: K. Kitamura et al., PloS one. 2011;6(11): e27706.

### Summary of the Invention

### Problem to be Solved by the Invention

It would be promising for regenerative medicine applications if cells producing the above growth factors could be induced or converted directly from ordinary somatic cells in a simple and rapid manner, but there is no known method for producing such cells.

The present invention primarily aims to provide a new method to safely and rapidly induce or convert growth factor-producing cells from somatic cells without the need for gene transfer. The present invention also aims to provide growth factor-producing cells useful in regenerative medicine, etc.

### Means for Solving the Problems

As a result of diligent study, the present inventors found that the use of TGF-β inhibitors, p53 inhibitors, cAMP inducers, and other agents can rapidly and directly convert somatic cells into growth factor-producing cells, and completed the present invention.

The present invention, for example, can include the following embodiments.
[1] A method for producing growth factor-producing cells by inducing differentiation directly from somatic cells, comprising a step of culturing a somatic cell in the presence of at least any one selected from the group consisting of a TGF-β inhibitor, a p53 inhibitor, and a cAMP inducer.
[2] The method for producing growth factor-producing cells according to the [1] above, wherein the step is a step of culturing a somatic cell in the presence of at least the following four kinds: a TGF-β inhibitor, a BMP inhibitor, a p53 inhibitor, and a cAMP inducer.
[3] The method for producing growth factor-producing cells according to the [1] or [2] above, wherein the step is a step of culturing a somatic cell in the presence of at least the following six kinds: a TGF-β inhibitor, a BMP inhibitor, a p53 inhibitor, a cAMP inducer, a GSK3 inhibitor, and an Erk inhibitor.
[4] The method for producing growth factor-producing cells according to the [2] or [3] above, wherein the TGF-β inhibitor is an ALK5 inhibitor and the BMP inhibitor is an ALK2/3 inhibitor.
[5] The method for producing growth factor-producing cells according to any one of the [1] to [4] above, wherein either the ALK5 inhibitor is SB431542 (CAS No.: 301836-41-9) or Repsox (CAS No.: 446859-33-2); or the ALK2/3 inhibitor is LDN193189 (CAS No.: 1062368-24-4); or the p53 inhibitor is pifithrin-α (CAS No.: 63208-82-2); or the cAMP inducer is forskolin (CAS No.: 66428-89-5).
[6] The method for producing growth factor-producing cells according to any one of the [3] to [5] above, wherein either the GSK3 inhibitor is CHIR99021 (CAS No.: 252917-06-9), or the Erk inhibitor is PD0325901 (CAS No.: 391210-10-9).
[7] The method for producing growth factor-producing cells according to any one of the [1] to [6] above, wherein the somatic cell is a terminally differentiated somatic cell.
[8] The method for producing growth factor-producing cells according to any one of the [1] to [7] above, wherein the somatic cell is a fibroblast.
[9] A growth factor-producing cell produced from the method for producing growth factor-producing cells according to any one of the [1] to [8] above.
[10] The growth factor-producing cell according to the [9] above, which produces a growth factor and an anti-inflammatory factor.
[11] The growth factor-producing cell according to the [10] above, wherein the growth factor is HGF and the anti-inflammatory factor is IL-1RN (Interleukin-1 receptor antagonist).
[12] A cell sheet or a pharmaceutical composition, comprising the growth factor-producing cell according to any one of the [9] to [11] above, and which is used for the treatment of a disease.
[13] The cell sheet or the pharmaceutical composition according to the [12] above, wherein the disease is a spinal cord injury, burn, or bedsore.
[14] A method for producing growth factors, comprising a step of extracting and isolating a growth factor from the growth factor-producing cell according to the [9] above or secretion thereof.
[15] A method for producing growth factors or anti-inflammatory factors, comprising a step of extracting and isolating a growth factor or an anti-inflammatory factor from the growth factor-producing cell according to the [10] or [11] above or secretion thereof.
[16] A composition for producing growth factor-producing cells by inducing differentiation directly from somatic cells, comprising at least any one selected from the group consisting of a TGF-β inhibitor, a p53 inhibitor, and a cAMP inducer.
[17] The composition according to the [16] above, comprising at least the following four kinds: a TGF-β inhibitor, a BMP inhibitor, a p53 inhibitor, and a cAMP inducer.
[18] The composition according to the [17] above, further comprising a GSK3 inhibitor and an Erk inhibitor.
[19] The composition according to the [17] or [18] above, wherein the TGF-β inhibitor is an ALK5 inhibitor and the BMP inhibitor is an ALK2/3 inhibitor.
[20] The composition according to any one of the [18] to [20] above, wherein either the ALK5 inhibitor is SB431542 (CAS No.: 301836-41-9) or Repsox (CAS No.: 446859-33-2); or the ALK2/3 inhibitor is LDN193189 (CAS No.: 1062368-24-4); or the p53 inhibitor is pifithrin-α (CAS No.: 63208-82-2); or the cAMP inducer is forskolin (CAS No.: 66428-89-5).
[21] The composition according to any one of the [18] to [20] above, wherein either the GSK3 inhibitor is CHIR99021 (CAS No.: 252917-06-9), or the Erk inhibitor is PD0325901 (CAS No.: 391210-10-9).

### Effect of the Invention

The present invention enables the production of growth factor-producing cells, growth factors, or anti-inflammatory factors from somatic cells (especially terminally differentiated somatic cells or fibroblasts) in a short period of time. The growth factor-producing cells obtained by the present invention are useful in regenerative medicine.

### Brief Description of the Drawings

[Figure 1] The upper figure shows phase contrast images of cells in fibroblast A47B22 (DMEM), and in NM and CiN cells derived from the same fibroblasts (day 8 of induction). The lower figure similarly shows a phase contrast image for KA45.
[Figure 2] The left figure shows HGF secretion in fibroblast A47B22 (DMEM), and in NM and CiN cells derived from the same fibroblasts (day 8 of induction). The right figure shows HGF secretion in fibroblast KA45 (DMEM), and in NM and CiN cells derived from the same fibroblast (day 8 of induction). In both cases, ND stands for Not Detected, which indicates that the levels were below the detection limit of the measurement method.
[Figure 3] The left figure shows IL-1RN secretion in fibroblasts A47B22 (DMEM), and in NM and CiN cells derived from the same fibroblasts (day 8 of induction). The right figure shows IL-1RN secretion in fibroblast KA45 (DMEM), and in NM and CiN cells derived from the same fibroblast (day 8 of induction). In both cases, ND stands for Not Detected, indicating that the levels were below the detection limit of the measurement method.
[Figure 4] The phase contrast images of cells at days 0, 4, 8, 12 and 16 of induction in NM (A47B22) and CiN cells (A47B22) derived from fibroblast A47B22, and in NM (KA45) and CiN cells (KA45) derived from fibroblast KA45 are shown.
[Figure 5] The changes in HGF secretion from day 0 to day 16 of induction in NM (A47B22) and CiN cells (A47B22) derived from fibroblast A47B22, and in NM(KA45) and CiN cells (KA45) derived from fibroblast KA45 are shown.
[Figure 6] The changes in IL-1RN secretion from day 0 to day 16 of induction in NM (A47B22) and CiN cells (A47B22) derived from fibroblast A47B22, and in NM (KA45) and CiN cells (KA45) derived from fibroblast KA45 are shown.
[Figure 7] The phase contrast images of fibroblast KA45-derived CiN cells (8 types) (day 8 of induction) cultured as Examples 2-1 to 2-8 are shown.
[Figure 8] The phase contrast images of fibroblast KA45-derived CiN cells (8 types) (day 8 of induction) cultured as Examples 2-9 to 2-16 are shown.
[Figure 9] HGF secretion in fibroblast KA45-derived CiN cells (8 types) (day 8 of induction) cultured as Examples 2-1 to 2-8 are shown.
[Figure 10] HGF secretion in fibroblast KA45-derived CiN cells (8 types) (day 8 of induction) cultured as Examples 2-9 to 2-16 are shown.
[Figure 11] IL-1RN secretion in fibroblast KA45-derived CiN cells (8 types) (day 8 of induction) cultured as Examples 2-1 to 2-8 are shown. ND stands for Not Detected, which indicates that the levels were below the detection limit of the measurement method.
[Figure 12] IL-1RN secretion in fibroblast KA45-derived CiN cells (8 types) (day 8 of induction) cultured as Examples 2-9 to 2-16 are shown. ND stands for Not Detected, which indicates that the levels were below the detection limit of the measurement method.
[Figure 13] HGF secretion in fibroblast KA45-derived CiN cells (14 types) (day 8 of induction) cultured as Examples 3-1 to 3-14 are shown.
[Figure 14] IL-1RN secretion in fibroblast KA45-derived CiN cells (14 types) (day 8 of induction) cultured as Examples 3-1 to 3-14 are shown. ND stands for Not Detected, which indicates that the levels were below the detection limit of the measurement method.

### Mode for Carrying Out the Present Invention

Hereinafter, the present invention will be described in detail.

### 1 Method for producing growth factor-producing cells

The method for producing growth factor-producing cells (hereinafter referred to as the "present invention production") is a method for producing growth factor-producing cells by inducing differentiation directly from somatic cells, which is characterized in a step of culturing a somatic cell in the presence of at least any one selected from the group consisting of a TGF-β inhibitor, a p53 inhibitor, and a cAMP inducer. The present invention production enables the production of cells that produce growth factors (e.g., HGF).

The step in the present invention production may include a step of culturing a somatic cell in the presence of at least the following four kinds: a TGF-β inhibitor, a BMP inhibitor, a p53 inhibitor, and a cAMP inducer. Furthermore, the step may also include a step of culturing a somatic cell in the presence of at least the following six kinds: a TGF-β inhibitor, a BMP inhibitor, a p53 inhibitor, a cAMP inducer, a GSK3 inhibitor, and an Erk inhibitor. Culturing a somatic cell in the presence of these four or six kinds allows the production of cells that produce not only growth factors but also anti-inflammatory factors (e.g., IL-1RN).

In the present invention production, the TGF-β inhibitor is preferably an ALK5 inhibitor, and the BMP inhibitor is preferably an ALK2/3 inhibitor. In the present invention production, it is more preferred that the ALK5 inhibitor is SB431542 (CAS No.: 301836-41-9) and/or Repsox (CAS No.: 446859-33- 2), and/or the ALK2/3 inhibitor is LDN193189 (CAS No.: 1062368-24-4) and/or dorsomorphin (CAS No.: 866405-64- 3). It is more preferred that the cAMP inducer is forskolin (CAS No.: 66428-89-5), the GSK3 inhibitor is CHIR99021 (CAS No.: 252917-06-9), the Erk inhibitor is PD0325901 (CAS No.: 391210-10-9), or the p53 inhibitor is pifithrin-α (CAS No.: 63208-82-2).

In the present invention production, somatic cells can be cultured in the presence of at least any one of the above inhibitors or inducers, and if necessary, other inhibitors or inducers can be optionally added to the somatic cells to produce growth factor-producing cells.

In each of the above inhibitors and inducers, one kind or a combination of two or more kinds may be used.

In the specific above inhibitors, etc., some may have more than one type of inhibitory action, etc., in which case a single inhibitor, etc. can be considered to be multiple inhibitors, etc.

The present invention production does not need to include a step of gene transfer. Here, "gene" refers to a substance (molecule or complex thereof) that has a chemical structure containing multiple nucleic acid bases and can be involved in carrying, transcription, or translation of genetic information or inhibition thereof, and includes both DNA and RNA. In addition, "gene transfer" includes the introduction of DNA by viral vectors, etc., as well as the introduction of RNA (siRNA, miRNA, piRNA, etc.) for the purpose of RNA silencing (RNA interference, etc.) and all other events in which a foreign gene is introduced into a cell. The present invention production allows growth factor-producing cells to be induced without gene transfer, and so the possibility (risk) of unexpected genomic mutation and insertion, as well as tumorigenesis, etc., can be made extremely low.

### 1.1 Somatic Cells

Cells of an organism can be classified into somatic and germ cells. Any somatic cell can be used as a starting material in the present invention production. The somatic cell is not particularly limited, and may be either a primary cell taken from a living body or a cell from an established cell line. Somatic cells at various stages of differentiation, e.g., terminally differentiated somatic cells, somatic cells on the way to terminal differentiation, or somatic cells that have been initialized to acquire pluripotency, can be used in the present invention production. The somatic cells that can be used in the present invention production include any somatic cells, for example, cells of the hematopoietic system (various lymphocytes, macrophages, dendritic cells, bone marrow cells, etc.), cells derived from organs (hepatocytes, splenocytes, pancreatic cells, kidney cells, lung cells, etc.), cells of the muscle tissue system (skeletal muscle cells, smooth muscle cells, myoblasts, cardiomyocytes, etc.), fibroblasts, neurons, osteoblasts, chondrocytes, endothelial cells, interstitial cells, adipocytes (white adipocytes, etc.), embryonic stem cells (ES cells), etc. The present invention production can also be applied to progenitor cells of these cells and cancer cells. Preferably, fibroblasts can be used.

Examples of the source of the above-mentioned somatic cells include, but are not limited to, humans, mammals other than humans, and animals other than mammals (birds, reptiles, amphibians, fishes, and the like). As a source of somatic cells, humans and mammals other than humans are preferred, and humans are particularly preferred. When growth factor-producing cells are produced by the present invention production for the purpose of administration to humans, preferably, somatic cells harvested from a donor having a matched or similar type of histocompatibility antigen to the recipient can be used. Somatic cells harvested from the recipient itself may be used for the production of growth factor-producing cells by the present invention production.

### 1.2 Inhibitors, etc. for the present invention

### 1.2.1 TGF-β inhibitors

TGF-β (transforming growth factor-β) has three types: TGF-β1, TGF-β2, and TGF-β3, and is produced in almost all cells. TGF-β is involved in a wide variety of cellular functions, including inhibition of cell proliferation, transformation, differentiation, development, and regulation of apoptosis in epithelial cells and many other cells.

In the above three types of TGF-β, ALK5 is also referred to as the TGF-β1 receptor. ALK5 is a serine/threonine kinase that forms a heterodimeric complex with the type II TGF-β receptor when bound to TGF-β, which transduces TGF-β signal from the cell surface to the cytoplasm.

In the present invention production, it is preferred that the TGF-β inhibitor be an ALK5 inhibitor. The term "in the presence of an ALK5 inhibitor" refers to culture conditions capable of inhibiting ALK5, and the means thereof is not particularly limited, and any means capable of inhibiting ALK5 can be used. Substances that directly act on ALK5 to inhibit its function (e.g., anti-ALK5 antibodies and other agents) and agents that inhibit the production of ALK5 itself can be used in the present invention. ALK5 can also be inhibited by inhibiting the signal transduction involving ALK5 upstream thereof.

Although not particularly limited in the present invention, examples of the ALK5 inhibitor can include the following compounds. Preferably, SB431542 or Repsox can be used.

### SB431542 (manufactured by Wako Pure Chemical Industries) (CAS No.: 301836-41-9)

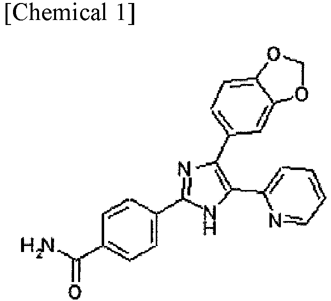

### ALK5 Inhibitor (Wako Pure Chemical Industries) or Repsox (manufactured by Abcam) (CAS No.: 446859-33-2)

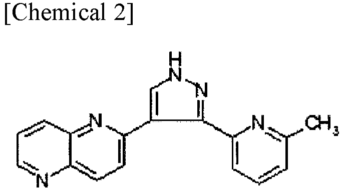

CultureSure (registered trademark) A83-01 (manufactured by Wako Pure Chemical Industries) (CAS No.: 909910-43-6)
D4476 (manufactured by Wako Pure Chemical Industries) (CAS No.: 301836-43-1)
LY364947 (manufactured by Wako Pure Chemical Industries) (CAS No.: 396129-53-6)
SB525334 (manufactured by Wako Pure Chemical Industries) (CAS No.: 356559-20-1)
SD208 (manufactured by Wako Pure Chemical Industries) (CAS No.: 627536-09-8)

The concentration of ALK5 inhibitor can be appropriately determined as needed and is not particularly limited, but can be used, for example, in the range of 0.2 µmol/L to 20 µmol/L, preferably 0.5 µmol/L to 10 µmol/L.

### 1.2.2 BMP inhibitors

Bone Morphogenetic Protein (BMP) is a member of the TGF-β superfamily of growth factors that regulates embryonic and tissue development, cell differentiation, and cell death. BMPs bind to type I and type II receptors on the cell membrane to form a heterotetramer that transduces BMP signals into the nucleus via phosphorylation of the transcription factor SMAD. Most BMP inhibitors inhibit phosphorylation of SMAD by ALK (Activin receptor-like kinase)-2,3,6, type I receptor activated by BMP binding.

Among the above ALKs, ALK2 is a receptor serine/threonine kinase of the ALK family members and is located upstream of signaling pathways involving SMAD proteins, especially SMAD1/5/8. The motility of prostate cancer cells is decreased by endoglin through activation of the ALK2-Smad1 pathway. The ALK2 gene is a major gene involved in progressive fibrodysplasia ossificans (FOP), a rare autosomal dominant congenital disorder characterized by progressive ectopic bone formation in muscle tissue.

ALK3 is a transmembrane serine/threonine kinase family member. The ALK3 gene acts as a minor susceptibility gene in PTEN (phosphatase and tensin homologue deleted on chromosome 10) mutation negative Cowden's disease. The ALK3 transport plays an important role in FOP lesion formation and is also involved in human T cell differentiation.

The term "in the presence of a BMP inhibitor" refers to culture conditions capable of inhibiting the BMP signaling pathway, and the means thereof is not particularly limited, and any means capable of inhibiting the BMP signaling pathway can be used. Substances that directly act on BMPs and BMP receptors to inhibit their functions (e.g., anti-BMP antibodies and other agents), or agents that inhibit their expression, can be used in the present invention. The BMP signaling pathway can also be inhibited by inhibiting the expression of SMAD transcription factors and their post-translational modifications, which are located downstream of the signaling involving BMPs.

In the present invention, somatic cells are cultured in the presence of BMP inhibitors, and among BMP inhibitors, it is preferable to culture somatic cells in the presence of ALK2/3 inhibitors.

The term "in the presence of an ALK2/3 inhibitor" refers to culture conditions capable of inhibiting both ALK2 and ALK3, and the means thereof is not particularly limited, and any means capable of inhibiting both ALK2 and ALK3 can be used. Substances that directly act on both ALK2 and ALK3 to inhibit their functions (e.g., anti-ALK2/3 antibodies and other agents) and agents that inhibit the production of ALK2 and ALK3 themselves can be used in the present invention. Provided that both ALK2 and ALK3 can be inhibited, it can also be done by inhibiting the signal transduction involving ALK2 and ALK3 upstream thereof.

Although the BMP inhibitors or ALK2/3 inhibitors that can be used in the present invention are not particularly limited as long as they are capable of performing their functions, and can include the following compounds. The preferable inhibitors can include LDN193189 and dorsomorphin, which are ALK2/3 inhibitors.

### LDN193189 (CAS No.: 1062368-24-4)

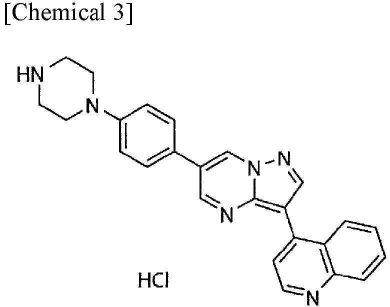

Dorsomorphin (AMPK Inhibitor, also called Compound C) (CAS No.: 866405-64-3)
Dorsomorphin dihydrochloride (CAS No.: 1219168-18-9)
DMH1 (CAS No.: 1206711-16-1)
K02288 (CAS No.: 1431985-92-0)
LDN212854 (CAS No.: 1432597-26-6)
LDN193189 HCl (CAS No.: 1062368-62-0)
MI,347 (CAS No.: 1062368-49-3)
LDN214117 (CAS No.: 1627503-67-6)

In the present invention, the concentration of BMP inhibitor or ALK2/3 inhibitor can be appropriately determined as needed and is not particularly limited, but can be used, for example, in the range of 0.1 µmol/L to 10 µmol/L, preferably 0.5 µmol/L to 5 µmol/L.

### 1.2.3 p53 inhibitors

p53 is one of the most important tumor suppressor genes, which inhibits cell proliferation and plays an important role in cancer suppression. It also activates target genes in response to various stresses and serves as a starting point for cell cycle arrest, apoptosis, DNA repair, and cellular senescence.

The term "in the presence of a p53 inhibitor" refers to culture conditions capable of inhibiting p53, and the means thereof is not particularly limited, and any means capable of inhibiting p53 can be used. Substances that directly act on p53 to inhibit its function (e.g., anti-p53 antibodies and other agents) and agents that inhibit the production of p53 itself can be used in the present invention. It is also possible to inhibit p53 by inhibiting signal transduction involving p53 upstream thereof.

Although the p53 inhibitors that can be used in the present invention are not particularly limited as long as they are capable of performing their function, and can include the following compounds. Preferably, pifithrin or pifithrin-α can be used.

### Pifithrin-α (CAS No.: 63208-82-2)

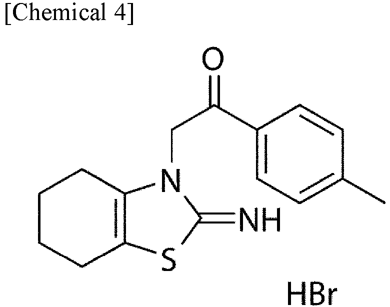

Pifithrin-β (CAS No.: 511296-88-1)
Pifithrin-µ (CAS No.: 64984-31-2)
NSC66811 (CAS No.: 6964-62-1)
Nultin-3 (CAS No.: 548472-68-0)

In the present invention, the concentration of p53 inhibitor can be appropriately determined as needed and is not particularly limited, but can be used, for example, in the range of 0.5 µmol/L to 30 µmol/L, preferably 1 µmol/L to 10 µmol/L.

### 1.2.4 cAMP inducer

Cyclic adenosine monophosphate (cAMP) is a second messenger that is involved in a variety of intracellular signaling events. The cAMP is produced intracellularly by cyclization of adenosine triphosphate (ATP) by adenylyl cyclase (adenylate cyclase).

The term "in the presence of a cAMP inducer" refers to culture conditions capable of inducing cAMP, and the means thereof is not particularly limited, and any means capable of increasing the intracellular cAMP level can be used. Adenylate cyclase activators such as substances that can induce cAMP by directly acting on adenylate cyclase, an enzyme involved in the generation of cAMP, substances that can promote the expression of adenylate cyclase, substances that inhibit phosphodiesterase, which is an enzyme that degrades cAMP, and other substances can be used to increase the intracellular cAMP concentration. It is also possible to use dibutyryl cAMP, which is a structural analogue of cAMP having the same effect as cAMP in cells.

Although the cAMP inducers that can be used in the present invention are not particularly limited as long as they are capable of performing their function, and can include forskolin (CAS No.: 66575-29-9) and forskolin derivatives (e.g., JP 2002-348243, A) and the following compounds. Preferably, forskolin can be used.

### Forskolin (CAS No.: 66428-89-5)

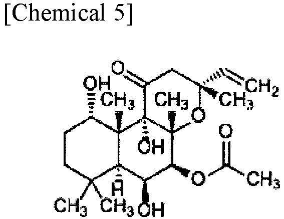

Isoproterenol (CAS No.: 7683-59-2)
NKH477 (CAS No.: 138605-00-2)
PACAP1-27 (CAS No.: 127317-03-7)
PACAP1-38 (CAS No.: 137061-48-4)

In the present invention, the concentration of the cAMP inducer can be appropriately determined as needed and is not particularly limited, but can be used, for example, in the range of 0.2 µmol/L to 50 µmol/L, preferably 1 µmol/L to 30 µmol/L.

### 1.2.5 GSK3 inhibitor

GSK3 (glycogen synthase kinase-3) was found as a protein kinase that phosphorylates and inactivates glycogen synthase. In mammals, GSK3 is classified into two isoforms, 51 kDa α (GSK3α) and 47 kDa β (GSK3β). GSK3 has the activity to phosphorylate various proteins and is involved in not only glycogen metabolism but also physiological phenomena such as cell division and cell proliferation.

The term "in the presence of GSK3 inhibitor" refers to culture conditions capable of inhibiting GSK3, and the means thereof is not particularly limited. Substances that inhibit GSK3 activity, such as anti-GSK3 antibodies and means inhibiting GSK3 signaling like GSK3 inhibitors, can be used. Since phosphorylation at a specific site of GSK3 causes its loss of activity, the means of promoting phosphorylation can also be used to inhibit GSK3 signaling.

The GSK3 inhibitors that can be used in the present invention are not particularly limited as long as they are capable of performing their function, and can include the following compounds. Preferably, the inhibitor can include CHIR99021.

### CHIR99021 (CAS No.: 252917-06-9)

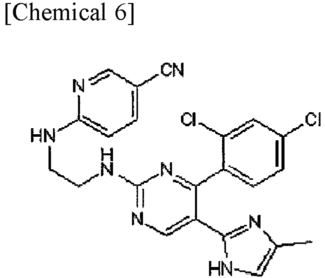

BIO((2'Z,3'E)-6-Bromoindirubin-3'-oxime) (CAS No.: 667463-62-9)
Kenpaullone (CAS No.: 142273-20-9)
A1070722 (CAS No.: 1384424-80-9)
SB216763 (CAS No.: 280744-09-4)
CHIR98014 (CAS No.: 556813-39-9)
TWS119 (CAS No.: 601514-19-6)
Tideglusib (CAS No.: 865854-05-3)
SB415286 (CAS No.: 264218-23-7)
Bikinin (CAS No.: 188011-69-0)
IM-12 (CAS No.: 1129669-05-1)
1-Azakenpaullone (CAS No.: 676596-65-9)
LY2090314 (CAS No.: 603288-22-8)
AZD1080 (CAS No.: 612487-72-6)
AZD2858 (CAS No.: 486424-20-8)
AR-A014418 (CAS No.: 487021-52-3)
TDZD-8 (CAS No.: 327036-89-5)
Indirubin (CAS No.: 479-41-4)

In the present invention, the concentration of GSK3 inhibitor can be appropriately determined as needed and is not particularly limited, but can be used, for example, in the range of 0.1 µmol/L to 20 µmol/L, preferably 0.5 µmol/L to 10 µmol/L.

### 1.2.6 Erk inhibitors

Erk is a subfamily of MAPKs that is activated by epidermal growth factor (EGF), serum stimulation, or oxidative stress, etc. Erk is divided into ERK1/2, ERK5, ERK7, and ERK8 based on the different signaling pathways involved. Signaling by ligand binding to tyrosine kinase receptors such as epidermal growth factor receptor (EGFR) results in phosphorylation of the TEY motif in the activation loop of Erk and Erk activation.

The term "in the presence of Erk inhibitor" refers to culture conditions capable of inhibiting Erk, and the means thereof is not particularly limited. Substances that inhibit the activity of Erk, such as anti-Erk antibodies and means inhibiting Erk signaling like Erk inhibitors, can be used. Means that inhibit enzymes involved in the activation of Erk, such as Erk kinase and Erk kinase kinase, can also be used for Erk inhibition.

Although the Erk inhibitors that can be used in the present invention are not particularly limited as long as they are capable of performing their function, and can include the following compounds. Preferably, the inhibitor can include PD0325901.

### PD0325901 (CAS No.: 391210-10-9)

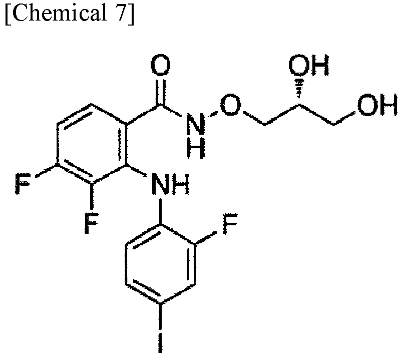

Olomoucine (CAS No.: 101622-51-9)
Aminopurvalanol A (CAS No.: 220792-57-4)
AS703026 (CAS No.: 1236699-92-5)
AZD8330 (CAS No.: 869357-68-6)
BIX02188 (CAS No.: 334949-59-6)
BIX02189 (CAS No.: 1265916-41-3)
CI-1040 (CAS No.: 212631-79-3)
Cobimetirlib (CAS No.: 934660-93-2)
GDC-0623 (CAS No.: 1168091-68-6)
MEK162 (CAS No.: 606143-89-9)
PD318088 (CAS No.: 391210-00-7)
PD98059 (CAS No.: 167869-21-8)
Refametinib (CAS No.: 923032-37-5)
RO4987655 (CAS No.: 874101-00-5)
SCH772984 (CAS No.: 942183-80-4)
Selumetinib (CAS No.: 606143-52-6)
SL327 (CAS No.: 305350-87-2)
Trametinib (CAS No.: 871700-17-3)
ARRY-142886 (CAS No.: 606143-52-6)
XI,518 (CAS No.: 934660-93-2)
RDEA119 (CAS No.: 923032-38-6)

In the present invention, the concentration of Erk inhibitor can be appropriately determined as needed and is not particularly limited, but can be used, for example, in the range of 0.1 µmol/L to 10 µmol/L, preferably 0.2 µmol/L to 5 µmol/L.

### 1.3 Other favorable conditions

Although not particularly limited in the present invention, it is preferable to culture somatic cells in the absence of histone-related components in the production step. The absence of components related to histone means the substantial absence of components related to histone, and include the case where components related to histone are present in trace amounts as well as the case where components related to histone are not present at all. Examples of histone-related components include histone deacetylase inhibitors (e.g., valproic acid). In the absence of histone deacetylase inhibitors, which are known to promote reprogramming by nuclear reprogramming factors, the risk of induction of pluripotent cells that may cause unintended differentiation is lower.

### 1.4 Somatic cell culture

Culturing somatic cells in the present invention production can be performed in the presence of the various inhibitors (and inducers or activators, as the case may be) described above, selecting the medium, temperature, and other conditions according to the type of somatic cells to be used. The culture medium can be selected from known or commercially available culture media. For example, MEM (minimum essential medium), DMEM (Dulbecco's modified Eagle's medium), DMEM/F12, or a modified version of these can be used with appropriate components (serum, proteins, amino acids, sugars, vitamins, fatty acids, antibiotics, etc.) added.

In the present invention, growth factor-producing cells are produced from somatic cells. Brain-derived neurotrophic factor (BDNF), glial cell-derived neurotrophic factor (GDNF), cAMP, ascorbic acid, ascorbic acid 2-phosphate, etc. are known to be effective substances for inducing differentiation into growth factor-producing cells. Substances that are commercially available as differentiation inducing agents, for example, can be used as substances effective in inducing differentiation into growth factor-producing cells. In the present invention, somatic cells may be cultured in the presence of the substances mentioned above.

For culture conditions, general cell culture conditions can be selected; conditions such as 37°C, 5% CO₂ are examples. During culture, the medium may preferably be changed at appropriate intervals (preferably once every 1 to 7 days, more preferably once every 3 to 4 days). When the present invention production is carried out using fibroblasts as material, growth factor-producing cells emerge in 4 days to 1 week under the conditions of 37°C, 5% CO₂. A culture period in the range of 4 to 16 days is appropriate. The range of 5 to 12 days is a preferable culture period, and the range of 7 to 9 days is a more preferable culture period.

Selection of somatic cells that are easy to culture makes it possible to increase the somatic cells in number in advance of converting them into growth factor-producing cells. Therefore, it is easy to produce growth factor-producing cells on a scaled-up basis.

Cell culture vessels such as plates, dishes, cell culture flasks, and cell culture bags can be used for culturing somatic cells. Gas permeable cell culture bags are suitable for cell culture. If a large number of cells are required, large culture vessels may be used. Culture can be performed in either an open or closed system, but when the obtained growth factor-producing cells are to be administered to humans, it is preferable to culture them in a closed system.

In the present invention production, culturing somatic cells in a medium containing various inhibitors, etc. mentioned above allows growth factor-producing cells to be produced from somatic cells in a single-step.

### 1.5 Growth factor-producing cells

The above-mentioned present invention production allows a cell population containing growth factor-producing cells to be obtained. The growth factor-producing cells produced by the present invention production also fall within the scope of the present invention (hereinafter referred to as the "present invention cell"). The present invention cell produces growth factors. The growth factors can include, for example, HGF (hepatocyte growth factor), NGF (nerve growth factor), VEGF (vascular endothelial growth factor), EGF (epidermal growth factor), etc. The present invention cell can be made to produce not only growth factors but also anti-inflammatory factors. The anti-inflammatory factors can include, for example, IL-1RN. Use of the above-mentioned present invention production allows the present invention cell to be produced by direct induction or direct conversion from somatic cells.

The present invention cell can be identified, for example, by morphological changes of the cells. Since growth factor-producing cells have a characteristic morphology depending on the cell type, the presence of growth factor-producing cells can be detected by comparing the morphology of the cells before and after culture. Growth factor-producing cells can also be identified by detecting molecules characteristic of growth factor-producing cells, such as enzymes, receptors, or low molecular weight compounds. Molecules characteristic of growth factor-producing cells include β3-tubulin, synapsin I, vesicular glutamate transporter (vGULT), microtubule-associated protein (MAP)2, γ-aminobutyric acid (GABA), tyrosine hydroxylase, and others, but are not limited to them.

Quarantine methods (detection with antibodies) can be used to detect the above molecules, and for protein molecules, the detection may be carried out by quantification of their mRNA levels. Antibodies that recognize molecules characteristic of growth factor-producing cells are also useful in isolating and purifying the present invention cell.

The present invention cell can be used for the treatment of diseases. In particular, it can be preferably applied to diseases for which the prognosis can be improved by promoting wound healing. For example, they are useful in the treatment of nervous system diseases, liver diseases, and skin diseases. In the treatment, the present invention cell may be transplanted directly into the affected area, or may be applied to the affected area in the form of cell sheets or pharmaceutical compositions, since cell sheets or pharmaceutical compositions can be produced using the present invention cell, as described below.

The above neurological diseases include spinal cord injury, cerebrovascular disorders (cerebral infarction, etc.), Parkinson's disease, Alzheimer's disease, amyotrophic lateral sclerosis, etc.

The above liver diseases include fatty liver, liver failure, hepatitis, etc.

The above skin diseases include burns, bedsores, etc.

Among the above diseases, the present invention cell can be used more preferably in the treatment of spinal cord injury, burns, and bedsores.

When the present invention cell is made into a cell sheet (hereinafter referred to as the "present invention cell sheet"), it may be made, for example, by coating a coatable substrate with a temperature-responsive substrate and culturing the present invention cell on the substrate thus coated in accordance with a conventional method.

As the temperature-responsive substrate, a temperature-responsive polymer grafted onto the cell culture substrate is appropriate. The material of the cell culture substrate is not particularly limited, but the cell attachment surface can be, for example, polystyrene, polycarbonate, or polymethyl methacrylate, or a combination of two or more of these. Among these, polystyrene is preferred.

The temperature-responsive polymers used in the present invention cell sheet may be homopolymers or copolymers. Monomers that can be used include acrylamide, N-alkyl substituted acrylamide derivatives, N,N-dialkyl substituted acrylamide derivatives, methacrylamide, N-alkyl substituted methacrylamide derivatives, N,N-dialkyl substituted methacrylamide derivatives, and vinylether derivatives. In the case of copolymers, any two or more of these can be used.

The method of coating the above-mentioned temperature-responsive substrate on the surface of the coatable substrate is not particularly restricted. For example, the above-mentioned cell culture substrate and the above-mentioned monomer or polymer can be coated on the surface of the coatable substrate by either of electron beam (EB) irradiation, electromagnetic wave irradiation (γ-ray irradiation, UV irradiation, etc.), plasma treatment, corona treatment, or organic polymerization reaction, or by physical adsorption such as coating or kneading.

The coatable substrates is appropriately glass, modified glass, polystyrene, polymethyl methacrylate, etc., which are usually used for cell culture. In addition to these, polymer compounds and inorganic compounds (ceramics, etc.) that can generally be given morphology can be widely used.

Detaching and collecting cultured cell sheets from the temperature-responsive substrate can be performed by setting the temperature of the culture substrate to which the cultured cells are attached above the upper critical solution temperature (UCST) or below the lower critical solution temperature (LCST) of the coating polymer (temperature-responsive polymer) on the culture substrate. The temperature change causes the coated polymer to change from a hydrophobic state to a hydrophilic state, allowing the cultured cells to be detached while maintaining cell-cell adhesion, and sheet-like cellular tissue can be recovered.

When the present invention cell is made into a pharmaceutical composition, the growth factor-producing cells may be mixed with a pharmaceutically acceptable carrier or the like in a form suitable for administration to an individual in accordance with a conventional method. The carrier can include, for example, physiological saline solution; and distilled water for injection which is made isotonic by the addition of glucose or other auxiliary agents (e.g., D-sorbitol, D-mannitol, sodium chloride, etc.). In addition, buffers (e.g., phosphate buffer, sodium acetate buffer), analgesics (e.g., benzalkonium chloride, procaine hydrochloride), stabilizers (e.g., human serum albumin, polyethylene glycol), preservatives, and antioxidants may be added.

The present invention cell can be further made into a composition in which other cells or components effective for the improvement of functionality and engraftment of growth factor-producing cells are combined.

Furthermore, the present invention cell can be used for screening of drug candidate compounds that act on growth factor-producing cells and for safety evaluation of drug candidate compounds. According to the present invention production, a large number of growth factor-producing cells can be obtained in a single operation, and thus the present invention production allows reproducible research results to be obtained without being affected by cell lot differences.

Treatment of the present invention cell with appropriate means allows growth factors (HGF, etc.) and anti-inflammatory factors (IL-1RN, etc.) to be extracted, isolated, and purified from the present invention cell or its secreted material. The appropriate means for the extraction/isolation can include conventional methods, and specifically include, for example, a method of dissolving the present invention cell in an appropriate surfactant; a freezing and thawing method by rapidly freezing a suspension of the present invention cell in liquid nitrogen and then thawing it; an osmotic shock method by suspending the present invention cell in a hypotonic solution such as sterile water; an ultrasonic treatment method by irradiating the suspension of the present invention cell with radiofrequency waves to disrupt the cell membrane; a mechanical method by crushing and grinding the present invention cell; and an enzymatic digestion method by treating the present invention cell with enzymes.

### 2 Composition

A composition in accordance with the present invention (hereinafter referred to as the "present invention composition") is a composition for producing growth factor-producing cells by inducing differentiation directly from somatic cells, and is characterized in comprising at least any one selected from the group consisting of a TGF-β inhibitor, a p53 inhibitor, and a cAMP inducer. Use of the present invention composition can produce the present invention cell. Use of the present invention composition enables the present invention cell to be produced by direct induction or direct conversion from somatic cells.

The present invention composition may be a composition comprising a step of culturing a somatic cell in the presence of at least the following four kinds: a TGF-β inhibitor, a BMP inhibitor, a p53 inhibitor, and a cAMP inducer. Furthermore, the present invention composition may be a composition comprising a GSK3 inhibitor and an Erk inhibitor in addition to at least any one of a TGF-β inhibitor, a p53 inhibitor, and a cAMP inducer or the four kinds mentioned above. Using the present invention composition comprising the above-mentioned four kinds, or six kinds of agents in which a GSK3 inhibitor and an Erk inhibitor are added to the four kinds allows the production of the present invention cell that can produce not only growth factors but also anti-inflammatory factors (e.g., IL-1RN).

In the present invention composition, the TGF-β inhibitor is preferably an ALK5 inhibitor, and the BMP inhibitor is preferably an ALK2/3 inhibitor. Also, in the present invention production, it is more preferable that the ALK5 inhibitor is SB431542 (CAS No.: 301836-41-9) and/or Repsox (CAS No.: 446859-33- 2), and/or the ALK2/3 inhibitor is LDN193189 (CAS No.: 1062368-24-4) and/or dorsomorphin (CAS No.: 866405-64- 3). It is also more preferable that the cAMP inducer is forskolin (CAS No.: 66428-89-5), the GSK3 inhibitor is CHIR99021 (CAS No.: 252917-06-9), the Erk inhibitor is PD0325901 (CAS No.: 391210-10-9), or p53 inhibitor is pifithrin-α (CAS No.: 63208-82-2).

The present invention composition is required to contain at least one of the above inhibitors, inducers, etc., and can optionally contain further inhibitors, inducers, etc., as needed.

In each of the above inhibitors and inducers, one kind or a combination of two or more kinds may be used.

In the specific inhibitors, etc. listed above, some may have more than one type of inhibitory action, etc., in which case, a single inhibitor, etc. can be considered to contain multiple inhibitors, etc.

The above specific and preferred examples of inhibitors, inducers, etc. are of the same meaning as the mentioned above.

The present invention composition can be used as a composition for producing growth factor-producing cells from somatic cells. The composition may also be used as a culture medium for producing growth factor-producing cells from somatic cells.

Examples of the mediums used for the production of growth factor-producing cells from somatic cells can include mediums containing an ALK5 inhibitor, which is a kind of TGF-β inhibitor, and a BMP inhibitor (preferably an ALK2/3 inhibitor), or in addition to these, any three or more selected from a group of four kinds consisting of a cAMP inducer, a GSK3 inhibitor, an Erk inhibitor, and a p53 inhibitor as active ingredients, in a basic medium made by mixing the ingredients necessary for cell culture. The above active ingredients need to be included only in a concentration effective for the production of growth factor-producing cells, and the concentration can be appropriately determined by those skilled in the art. The basic medium can be selected from known or commercially available culture media. For example, the common media MEM (Minimum Essential Medium), DMEM (Dulbecco's Modified Eagle's Medium), DMEM/F12, or modified versions of these media can be used as the basic medium.

The medium may be further supplemented with the known medium components mentioned above herein, such as serum, proteins (albumin, transferrin, growth factors, etc.), amino acids, sugars, vitamins, fatty acids, antibiotics, etc.

The medium may also be supplemented with substances effective in inducing differentiation into growth factor-producing cells, such as brain-derived neurotrophic factor (BDNF), glial cell-derived neurotrophic factor (GDNF), cAMP, ascorbic acid, ascorbate 2-phosphate, etc., as mentioned above in the description.

Furthermore, in the present invention, administering a composition containing, for example, an ALK5 inhibitor, which is a kind of TGF-β inhibitor and a BMP inhibitor (preferably an ALK2/3 inhibitor), or in addition to these, any three or more selected from the group of four kinds consisting of a cAMP inducer, a GSK3 inhibitor, an Erk inhibitor, and a p53 inhibitor to a living body, also allows growth factor-producing cells to be produced from somatic cells in vivo. That is, according to the present invention, a method for producing growth factor-producing cells from somatic cells in vivo is provided, wherein the method comprises administering to a living body a composition containing an ALK5 inhibitor, which is a kind of TGF-β inhibitor and a BMP inhibitor (preferably an ALK2/3 inhibitor), or in addition to these, any three or more selected from the group of four kinds consisting of a cAMP inducer, a GSK3 inhibitor, an Erk inhibitor, and a p53 inhibitor. Preferred combinations of the inhibitors and the like to be administered to a living body are as described herein.

Examples of the living body include humans, mammals other than humans, and animals other than mammals (birds, reptiles, amphibians, fish, etc.), but humans are particularly preferred. For example, administering a composition containing an ALK5 inhibitor, which is a kind of TGF-β inhibitor and a BMP inhibitor (preferably an ALK2/3 inhibitor), or in addition to these, any three or more selected from the group of four kinds consisting of a cAMP inducer, a GSK3 inhibitor, an Erk inhibitor, and a p53 inhibitor to a specific site of a living body, allows growth factor-producing cells to be produced from somatic cells, in the specific site mentioned above.

### EXAMPLES

Hereinafter, the present invention will be specifically illustrated by way of Examples, but the present invention is not limited to the scope of the Examples.

### [Example 1] Production of growth factor-producing cells

### (1) Human fibroblasts

Background information on the two lines of human fibroblasts used as material is shown in Table 1.

**Table 1]**

| NAME | Company | Lot# | Passage | Donor Ages | Gender | BMI | Depot |
|---|---|---|---|---|---|---|---|
| A47B22 | Zenbio | DFM092514B | 15-20 | 47 | F | 21.9 | Abdomen |
| KA45 | LIFELINE | 3761 | 8-10 | 45 | F | - | Abdomen |

### (2) Direct induction of cytokine-secreting cells from human fibroblasts

The human fibroblasts listed in Table 1 were seeded at 1 × 10⁵ cells per 35-mm dish or per well in a 6-well plate, respectively, and they were incubated under the conditions of 37°C, 5%CO₂ for 2 days in DMEM high glucose culture solution supplemented with 10% FBS, 100 U/mL penicillin, and 100 µg/mL streptomycin. Each cell line was subjected to culture in the range of passages as defined in Table 1.

Neuronal cell medium containing the seven low-molecular-weight compounds listed in Table 2 was prepared. This neuronal cell medium was a 1:1 mixture of DMEM/F12 containing 1% (v/v) N2 supplement (Lifetech) and NeurobasalMedium (Lifetech) containing 2% (v/v) B27 supplement (Lifetech). The above-mentioned medium in the dish of human fibroblasts cultured for 2 days was replaced with this neuronal cell medium, and the culture was continued while changing the medium every 2 or 3 days under the conditions of 37°C, 5%CO₂.

Human fibroblasts cultured for 2 days are called DMEM, cells cultured with replacement of medium to a neuronal cell medium are called NM, and cells cultured in a neuronal cell medium containing the 7 low-molecular-weight compounds are called CiN.

**Table 2**

| Low molecular weight compound | Classification | Final conc. | Cat No. | mfr. |
|---|---|---|---|---|
| CHIR99021 (G) | GSK3 inhibitor | 1 µM | CAY-13122 | Cayman Chemical |
| PD0325901 (M) | Erk inhibitor | 1 µM | 168-25293 | Wako |
| LDN193189 | ALK2/3 inhibitor | 1 µM | SML0559 | SIGMA |
| Pifithrin-α | p53 inhibitor | 5 µM | 162-23133 | Wako |
| Forskolin | cAMP inducer | 7.5 µM | 063-02193 | Wako |
| SB431542 | ALK5 inhibitor | 2 µM | 198-16543 | Wako |
| Dorsomorphin | ALK2/3/6 inhibitor | 1 µM | 040-33753 | Wako |

### (3) Evaluation 1 of cytokine-secreting cells

Culture supernatants (collected in 24 hours) of DMEM, and 8-day-induced NM and CiN were collected and the secreted levels of cytokines HGF and IL-1RN were measured by ELISA. The ELISA kits used for the measurements were as follows.
AuthentiKine^{™} Human HGF ELISA Kit (Proteintech, Cat No. # PGI-KE00168-1)
Human IL-1ra/IL-1F3 Quantikine ELISAKit (R&D, Cat No. #DRA00B)

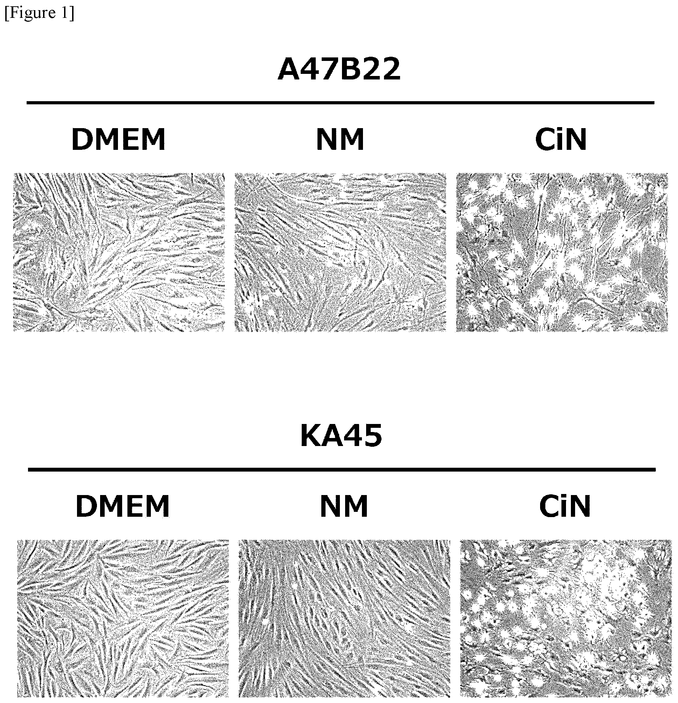

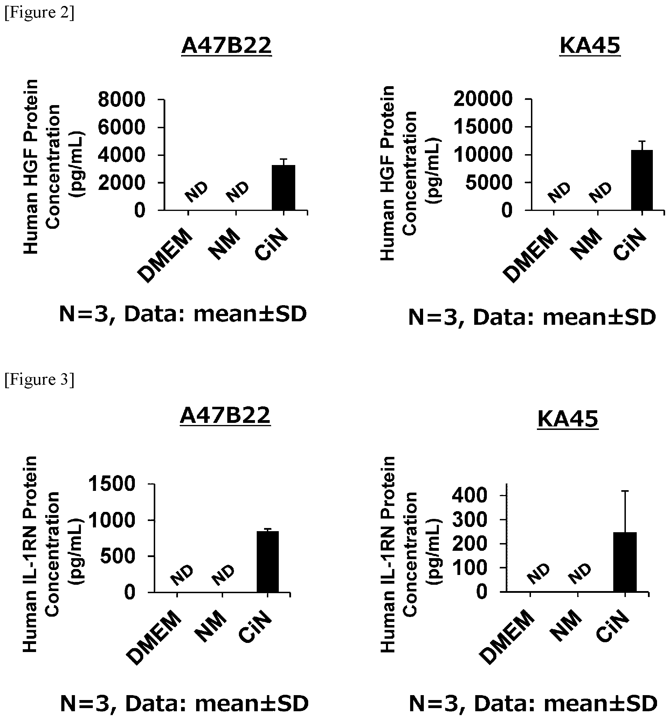

DMEM, and 8-day-induced NM and CiN are shown in Figure 1. The results of measurement of the secreted levels of HGF and IL-1RN are shown in Figures 2 and 3, respectively. It can be seen that CiN secretes both HGF and IL-1RN.

### (4) Evaluation 2 of cytokine-secreting cells

Culture supernatants (collected in 24 hours) of NM and CiN induced for 0, 4, 8, 12 and 16 days were collected and the secreted levels of cytokines HGF and IL-1RN were measured by ELISA. The ELISA kits used for the measurements were as follows.
AuthentiKine^{™} Human HGF ELISA Kit (Proteintech, Cat No. # PGI-KE00168-1)
Human IL-1ra/IL-1F3 Quantikine ELISAKit (R&D, Cat No. #DRA00B)

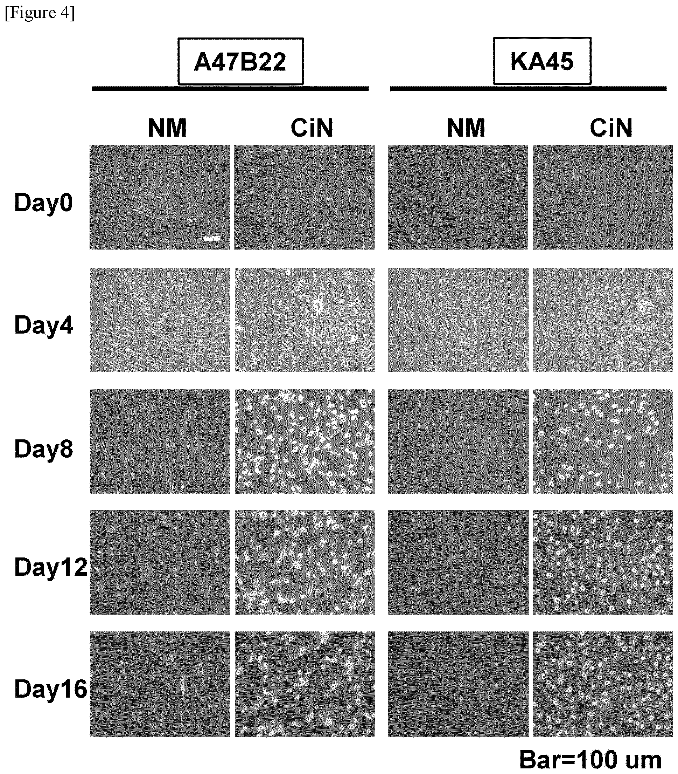

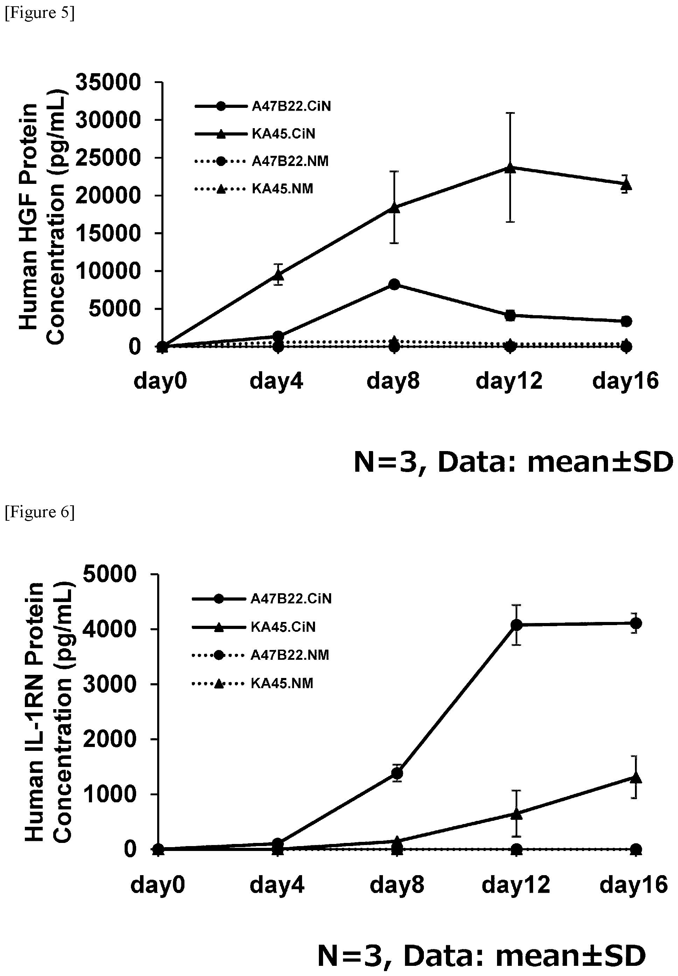

The appearances of the NM and CiN inductions are shown in Figure 4. The results of measurement of the secreted levels of HGF and IL-1RN are also shown in Figures 5 and 6, respectively. It can be seen that CiN secretes both HGF and IL-1RN well, especially around or after Day 8.

### [Example 2]

### (1) Direct induction of cytokine-secreting cells from human fibroblasts (2)

Human fibroblasts KA45 shown in Table 1 were seeded at 1 × 10⁵ cells per 35-mm dish or per well in a 6-well plate, and they were incubated under the conditions of 37°C, 5%CO₂ for 2 days in DMEM high glucose culture solution supplemented with 10% FBS, 100 U/mL penicillin, and 100 µg/mL streptomycin. The cell line was subjected to culture in the range of passages as defined in Table 1.

Neuronal cell medium containing the seven low-molecular-weight compounds listed in Table 2 or Table 3 and Neuronal cell mediums lacking either one of these seven low-molecular-weight compounds were prepared. The neuronal cell mediums were a 1:1 mixture of DMEM/F12 containing 1% (v/v) N2 supplement (Lifetech) and NeurobasalMedium (Lifetech) containing 2% (v/v) B27 supplement (Lifetech). The above-mentioned medium in the dish of human fibroblasts cultured for 2 days was replaced with this neuronal cell medium, and the culture was continued while changing the medium every 2 or 3 days under the conditions of 37°C, 5%CO₂.

**[Table 3]**

| Low molecular weight compound | Classification | Final conc. | Cat No. | mfr. |
|---|---|---|---|---|
| CHIR99021 (G) | GSK3 inhibitor | 1 µM | CAY-13122 | Cayman Chemical |
| PD0325901 (M) | Erk inhibitor | 1 µM | 168-25293 | Wako |
| LDN193189 | ALK2/3 inhibitor | 1 µM | SML0559 | SIGMA |
| Pifithrin-α | p53 inhibitor | 5 µM | 162-23133 | Wako |
| Forskolin | cAMP inducer | 7.5 µM | 063-02193 | Wako |
| Dorsomorphin | ALK2/3/6 inhibitor | 1 µM | 040-33753 | Wako |
| Repsox | ALK5 inhibitor | 2 µM | ab142139 | Abcam |

**[Table 4]**

| | Ex.2-1 | Ex.2-2 | Ex.2-3 | Ex.2-4 | Ex.2-5 | Ex.2-6 | Ex.2-7 | Ex.2-8 |
|---|---|---|---|---|---|---|---|---|
| SB431542 | ○ | - | ○ | ○ | ○ | ○ | ○ | ○ |
| LDN193189 | ○ | ○ | - | ○ | ○ | ○ | ○ | ○ |
| Dorsomorphin | ○ | ○ | ○ | - | ○ | ○ | ○ | ○ |
| Pifithrin-α | ○ | ○ | ○ | ○ | - | ○ | ○ | ○ |
| CHIR99021 (G) | ○ | ○ | ○ | ○ | ○ | - | ○ | ○ |
| PD0325901 (M) | ○ | ○ | ○ | ○ | ○ | ○ | - | ○ |
| Forskolin | ○ | ○ | ○ | ○ | ○ | ○ | ○ | - |

In the table, "Ex." means Example."

**[Table 5]**

| | Ex.2-9 | Ex.2-10 | Ex.2-11 | Ex.2-12 | Ex.2-13 | Ex.2-14 | Ex.2-15 | Ex.2-16 |
|---|---|---|---|---|---|---|---|---|
| Repsox | ○ | - | ○ | ○ | ○ | ○ | ○ | ○ |
| LDN193189 | ○ | ○ | - | ○ | ○ | ○ | ○ | ○ |
| Dorsomorphin | ○ | ○ | ○ | - | ○ | ○ | ○ | ○ |
| Pifithrin-α | ○ | ○ | ○ | ○ | - | ○ | ○ | ○ |
| CHIR99021 (G) | ○ | ○ | ○ | ○ | ○ | - | ○ | ○ |
| PD0325901 (M) | ○ | ○ | ○ | ○ | ○ | ○ | - | ○ |
| Forskolin | ○ | ○ | ○ | ○ | ○ | ○ | ○ | - |

In the table, "Ex." means Example."

### (2) Evaluation of cytokine-secreting cells

Culture supernatants (collected in 24 hours) of cells induced for 8 days were collected and the secreted levels of the cytokines HGF and IL-1RN were measured by ELISA. The ELISA kits used for the measurements were as follows.
AuthentiKine^{™} Human HGF ELISA Kit (Proteintech, Cat No. # PGI-KE00168-1)
Human IL-1ra/IL-1F3 Quantikine ELISAKit (R&D, Cat No. #DRA00B)

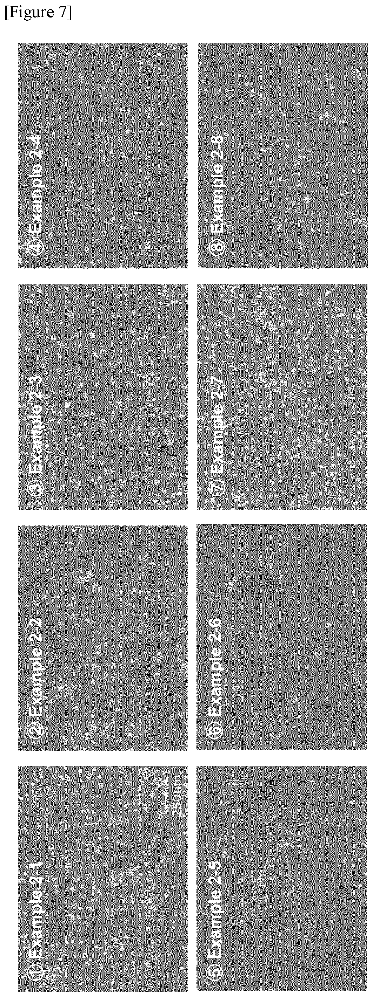

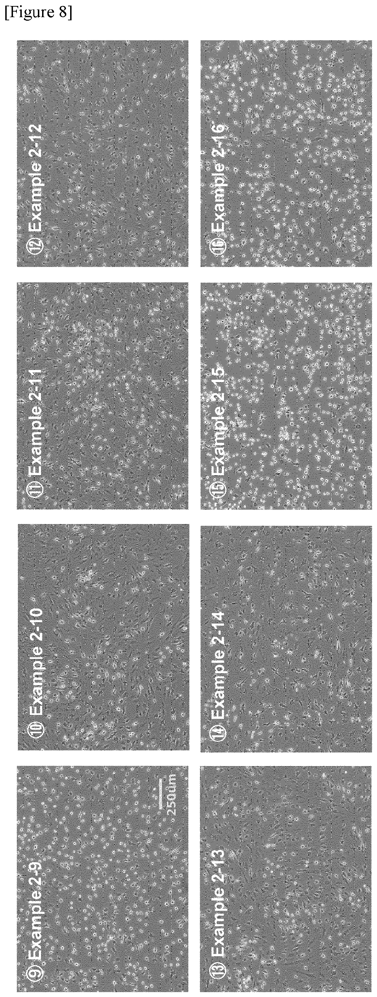

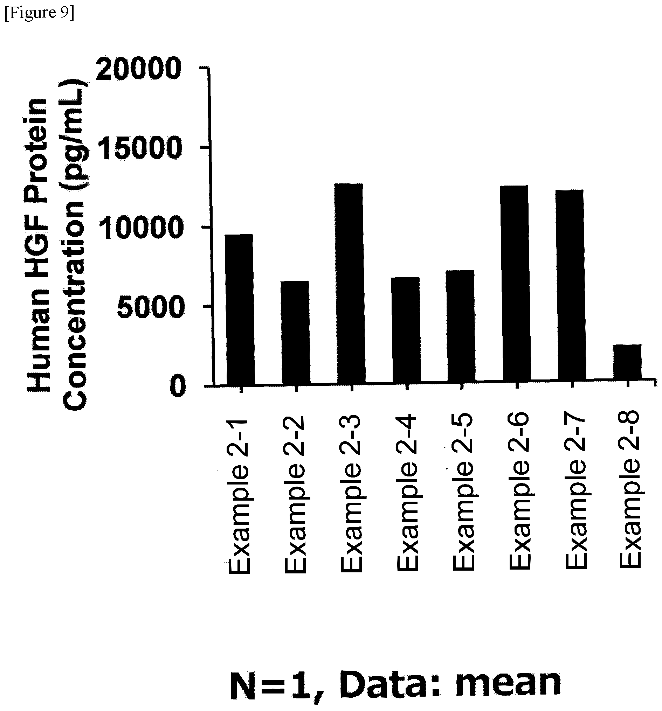

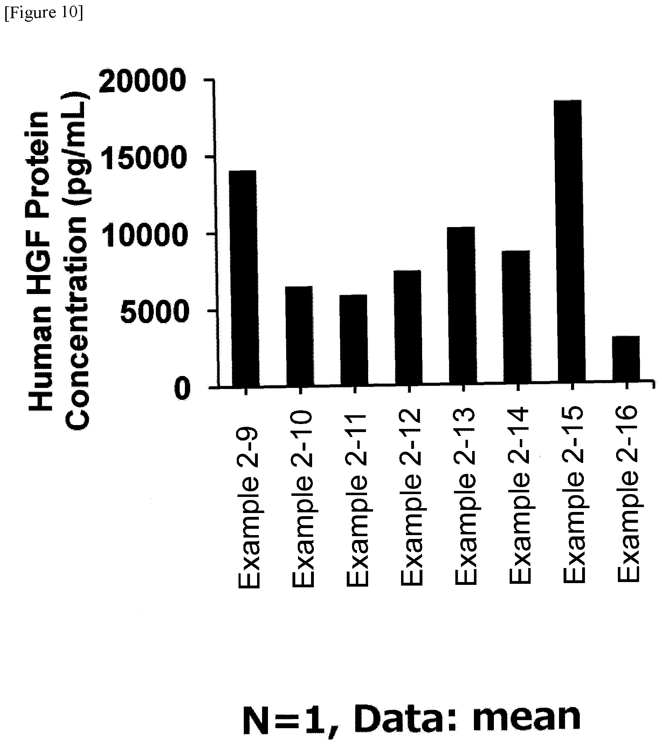

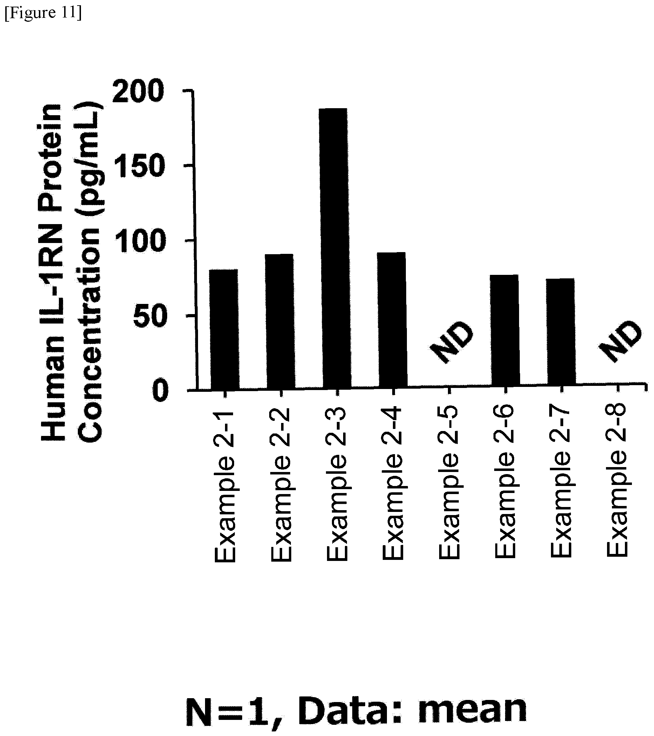

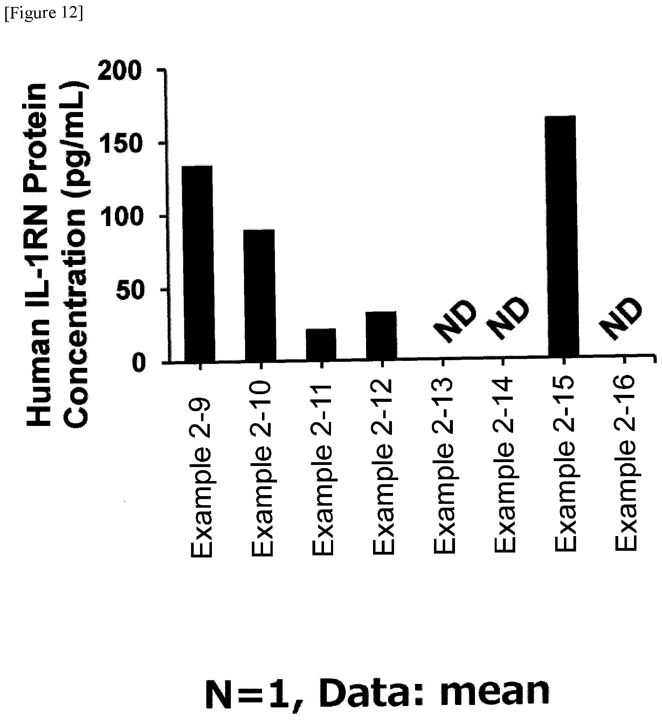

Each cell line induced for 8 days (Examples 2-1 to 2-16) is shown in Figures 7 and 8. The results of measurement of the secreted levels of HGF are shown in Figures 9 and 10, and those of IL-1RN are shown in Figures 11 and 12. HGF were secreted well in all Examples. While, IL-1RN were secreted well only in Examples in which the induction was carried out using at least the following four kinds: a TGF-β inhibitor, a BMP inhibitor, a p53 inhibitor, and a cAMP inducer.

### [Example 3]

### (1) Direct induction of cytokine-secreting cells from human fibroblasts (3)

Human fibroblasts KA45 shown in Table 1 were seeded at 1 × 10⁵ cells per 35-mm dish or per well in a 6-well plate, and they were incubated under the conditions of 37°C, 5%CO₂ for 2 days in DMEM high glucose culture solution supplemented with 10% FBS, 100 U/mL penicillin, and 100 µg/mL streptomycin. Each cell line was subjected to culture in the range of passages as defined in Table 1. Neuronal cell medium containing the seven low-molecular-weight compounds listed in Table 2 and neuronal cell mediums lacking several of these seven low-molecular-weight compounds were prepared. The combinations of low-molecular weight compounds used are listed in Table 6.

**[Table 6]**

| | Ex. 3-1 | Ex. 3-2 | Ex. 3-3 | Ex. 3-4 | Ex. 3-5 | Ex. 3-6 | Ex. 3-7 | Ex. 3-8 | Ex. 3-9 | Ex. 3-10 | Ex. 3-11 | Ex. 3-12 | Ex. 3-13 | Ex. 3-14 |
|---|---|---|---|---|---|---|---|---|---|---|---|---|---|---|
| SB431542 | ○ | ○ | ○ | ○ | ○ | ○ | ○ | - | ○ | - | - | ○ | ○ | ○ |
| LDN193189 | ○ | ○ | ○ | - | - | - | - | - | - | - | - | ○ | ○ | ○ |
| Dorsomorphin | ○ | ○ | - | ○ | - | - | - | - | - | - | - | - | - | - |
| Pifithrin-α | ○ | ○ | ○ | ○ | ○ | - | ○ | ○ | - | ○ | - | ○ | ○ | ○ |
| CHIR99021 (G) | ○ | - | - | - | - | - | - | - | - | - | - | ○ | - | - |
| PD0325901 (M) | ○ | - | - | - | - | - | - | - | - | - | - | ○ | - | ○ |
| Forskolin | ○ | ○ | ○ | ○ | ○ | ○ | - | ○ | - | - | ○ | ○ | ○ | ○ |

In the table, "Ex." means Example."

The neuronal cell mediums were a 1:1 mixture of DMEM/F12 containing 1% (v/v) N2 supplement (Lifetech) and NeurobasalMedium (Lifetech) containing 2% (v/v) B27 supplement (Lifetech). The above-mentioned medium in the dish of human fibroblasts cultured for 2 days was replaced with this neuronal cell medium, and the culture was continued while changing the medium every 2 or 3 days under the conditions of 37°C, 5%CO₂.

### (2) Evaluation of cytokine-secreting cells

Culture supernatants (collected in 24 hours) of cells induced for 8 days were collected and the secreted levels of the cytokines HGF and IL-1RN were measured by ELISA. The ELISA kits used for the measurements were as follows.
AuthentiKine^{™} Human HGF ELISA Kit (Proteintech, Cat No. # PGI-KE00168-1)
Human IL-1ra/IL-1F3 Quantikine ELISAKit (R&D, Cat No. #DRA00B)

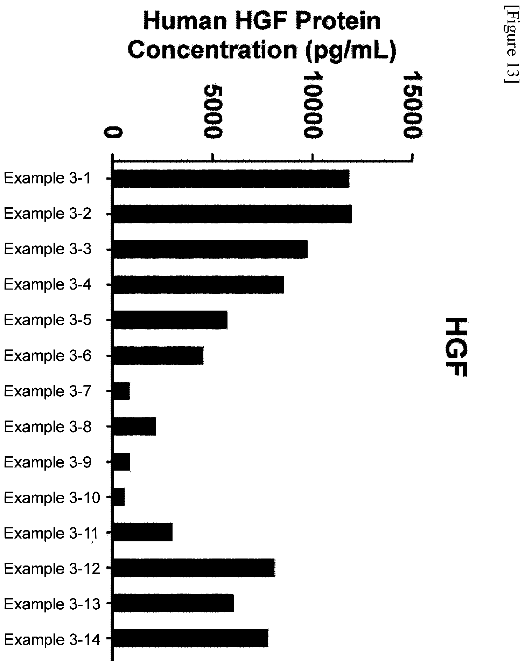

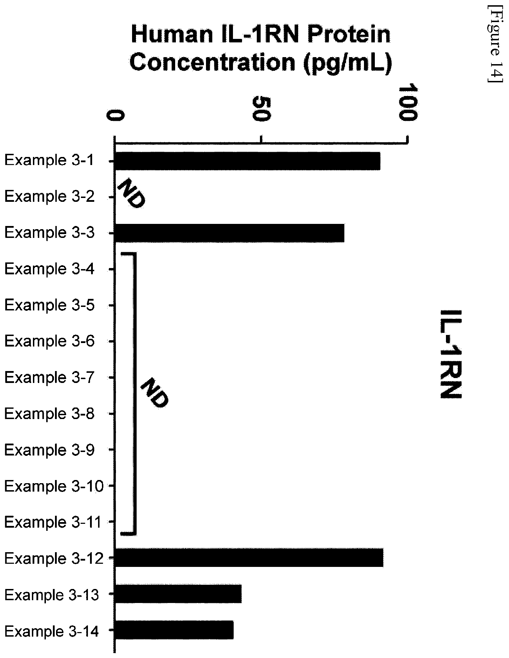

For each of the cells induced for 8 days (Examples 3-1 to 3-14), the results of measurement of the secreted levels of HGF are shown in Figure 13, and those of IL-1RN are shown in Figure 14. HGF were secreted well in all Examples. While, IL-1RN were secreted well only in Examples in which the induction was carried out using at least the following four kinds: aTGF-β inhibitor, a BMP inhibitor, a p53 inhibitor, and a cAMP inducer. Furthermore, both HGF and IL-1RN were well secreted in the cases where the induction was carried out using a TGF-β inhibitor, a BMP inhibitor, a p53 inhibitor, a cAMP inducer, a GSK3 inhibitor, and an Erk inhibitor.

### [Industrial applicability]

The present invention allows the production of cells that produce growth factors (especially HGF) or cells that further produce anti-inflammatory factors (especially IL-1RN) as well from somatic cells (especially terminally differentiated somatic cells or fibroblasts) in a short period of time. Cells produced from the present invention are useful in medical treatment related to regeneration and wound healing promotion for spinal cord injury, burns, bedsores, etc.

## Claims

1. A method for producing growth factor-producing cells by inducing differentiation directly from somatic cells, comprising a step of culturing a somatic cell in the presence of at least any one selected from the group consisting of a TGF-β inhibitor, a p53 inhibitor, and a cAMP inducer.

2. The method for producing growth factor-producing cells according to claim 1, wherein the step is a step of culturing a somatic cell in the presence of at least the following four kinds: a TGF-β inhibitor, a BMP inhibitor, a p53 inhibitor, and a cAMP inducer.

3. The method for producing growth factor-producing cells according to claim 1 or 2, wherein the step is a step of culturing a somatic cell in the presence of at least the following six kinds: a TGF-β inhibitor, a BMP inhibitor, a p53 inhibitor, a cAMP inducer, a GSK3 inhibitor, and an Erk inhibitor.

4. The method for producing growth factor-producing cells according to claim 2 or 3, wherein the TGF-β inhibitor is an ALK5 inhibitor and the BMP inhibitor is an ALK2/3 inhibitor.

5. The method for producing growth factor-producing cells according to any one of claims 1 to 4, wherein either the ALK5 inhibitor is SB431542 (CAS No.: 301836-41-9) or Repsox (CAS No.: 446859-33-2); or the ALK2/3 inhibitor is LDN193189 (CAS No.: 1062368-24-4); or the p53 inhibitor is pifithrin-α (CAS No.: 63208-82-2); or the cAMP inducer is forskolin (CAS No.: 66428-89-5).

6. The method for producing growth factor-producing cells according to any one of claims 3 to 5, wherein either the GSK3 inhibitor is CHIR99021 (CAS No.: 252917-06-9), or the Erk inhibitor is PD0325901 (CAS No.: 391210-10-9).

7. The method for producing growth factor-producing cells according to any one of claims 1 to 6, wherein the somatic cell is a terminally differentiated somatic cell.

8. The method for producing growth factor-producing cells according to any one of claims 1 to 7, wherein the somatic cell is a fibroblast.

9. A growth factor-producing cell produced from the method for producing growth factor-producing cells according to any one of claims 1 to 8.

10. The growth factor-producing cell according to claim 9, which produces a growth factor and an anti-inflammatory factor.

11. The growth factor-producing cell according to claim 10, wherein the growth factor is HGF and the anti-inflammatory factor is IL-1RN (Interleukin-1 receptor antagonist).

12. A cell sheet or a pharmaceutical composition, comprising the growth factor-producing cell according to any one of claims 9 to 11, and which is used for the treatment of a disease.

13. The cell sheet or the pharmaceutical composition according to claim 12, wherein the disease is a spinal cord injury, burn, or bedsore.

14. A method for producing growth factors, comprising a step of extracting and isolating a growth factor from the growth factor-producing cell according to claim 9 or secretion thereof.

15. A method for producing growth factors or anti-inflammatory factors, comprising a step of extracting and isolating a growth factor or an anti-inflammatory factor from the growth factor-producing cell according to claim 10 or 11 or secretion thereof.

16. A composition for producing growth factor-producing cells by inducing differentiation directly from somatic cells, comprising at least any one selected from the group consisting of a TGF-β inhibitor, a p53 inhibitor, and a cAMP inducer.

17. The composition according to claim 16, comprising at least the following four kinds: a TGF-β inhibitor, a BMP inhibitor, a p53 inhibitor, and a cAMP inducer.

18. The composition according to claim 17, further comprising a GSK3 inhibitor and an Erk inhibitor.

19. The composition according to claim 17 or 18, wherein the TGF-β inhibitor is an ALK5 inhibitor and the BMP inhibitor is an ALK2/3 inhibitor.

20. The composition according to any one of claims 18 to 20, wherein either the ALK5 inhibitor is SB431542 (CAS No.: 301836-41-9) or Repsox (CAS No.: 446859-33-2); or the ALK2/3 inhibitor is LDN193189 (CAS No.: 1062368-24-4); or the p53 inhibitor is pifithrin-α (CAS No.: 63208-82-2); or the cAMP inducer is forskolin (CAS No.: 66428-89-5).

21. The composition according to any one of claims 18 to 20, wherein either the GSK3 inhibitor is CHIR99021 (CAS No.: 252917-06-9), or the Erk inhibitor is PD0325901 (CAS No.: 391210-10-9).
